# EUROPEAN PATENT APPLICATION

(11) **EP 3 480 196 A1**
(43) Date of publication of application: **08.05.2019**
(21) Application number: 17199710.9
(22) Date of filing: 02.11.2017
(51) Int. Cl.: C07D 417/06, A01N 55/00

(54) **PROCESS FOR THE PREPARATION OF THIAMETHOXAM**

(71) Applicant: Jiangsu Rotam Chemistry Co., Ltd, Kunshan, Jiangsu 215301 (CN)
(72) Inventor: BRISTOW, James Timothy, Chai Wan, Hong Kong N/A (CN)
(74) Representative: Akers, Noel James

(57) **Abstract**

There is disclosed the use of a solvent system comprising a C₁ to C₄ alcohol, a substituted benzene derivative bearing two or more C₁ to C₄ alkyl substituents, or a mixture thereof in the preparation of a crystalline form of 3-(2-chloro-1,3-thiazol-5-yl methyl)-5-methyl-1,3,5-oxadiazinan-4-yledene (nitro)amine (thiamethoxam). Crystalline thiamethoxam prepared in this way exhibits a significantly improved stability, in particular a resistance to photolysis, compared with crystalline thiamethoxam prepared using other solvents.

## Description

The present invention relates to the preparation of 3-(2-chloro-1,3-thiazol-5-ylmethyl)-5-methyl-1,3,5-oxadiazinan-4-yledene(nitro)amine (thiamethoxam).

3-(2-chloro-1,3-thiazol-5-ylmethyl)- 5-methyl-1,3,5- oxadiazinan-4-yledene(nitro)amine, known by the common name thiamethoxam, is a widely used commercial agrochemical product exhibiting insecticidal activity. Thiamethoxam was first disclosed in EP 0 580 553, along with methods for its preparation.

Thiamethoxam was first marketed in 1991 and is a systemic insecticide that is absorbed quickly by plants and transported to all parts of the plant, where it acts as a deterrent to insect feeding. Thiamethoxam is active in the stomach of the insects and also acts through direct contact. The compound interferes with information transfer between nerve cells, in turn paralyzing the insects. Thiamethoxam is effective against aphids, thrips, beetles, centipedes, millipedes, sawflies, leaf miners, stem borers and termites. It has also been asserted that thiamethoxam triggers various physiological reactions, which induce the expression of specific functional proteins involved in various stress defense mechanisms of the plant allowing it to better cope under tough growing conditions.

Thiamethoxam is a moderately toxic substance. In normal use, there are no unacceptable risks involved with its application as an insecticide. The substance is toxic to bees and harmful to aquatic and soil organisms, although the level of toxicity to bees is not yet completely clear. A metabolite of thiamethoxam in soil is clothianidin.

There are several ways of preparing thiamethoxam known and reported in the art. A number of methods are based on the reaction of 2-(phenylthio)-5-chloromethyl-thiozole and 3-methyl-4-nitroiminoperhydro-1,3,5-oxadiazine, followed by chlorination of the resulting compound. The general procedure of this reaction sequence is as follows:

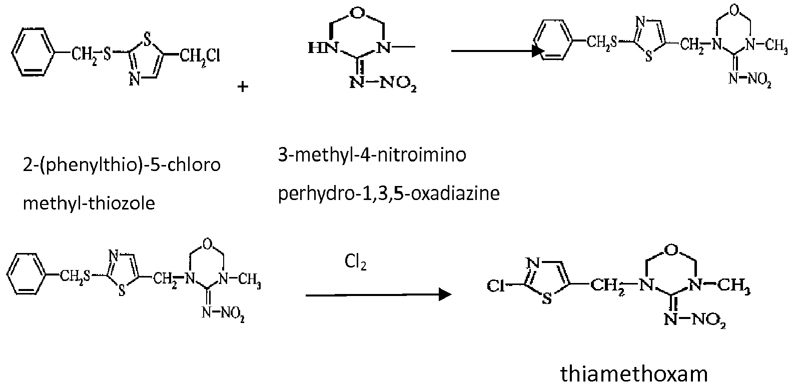

EP 1 187 833 discloses a method for preparing thiamethoxam by reacting 3-methyl-N-nitro-1,3,5, oxadiazinan-4-imine and 2-chloro-5-chloromethyl thiazole in a solvent of ester of carbonic acid. However, the yield and level of purity is not satisfactory.

A similar reaction scheme is disclosed by P. Maienfisch Synthesis and Properties of Thiamethoxam and Related Compounds', Z. Naturforsch, 61b, (2006), pages 353 to 359. The reaction sequence for this method is as follows:

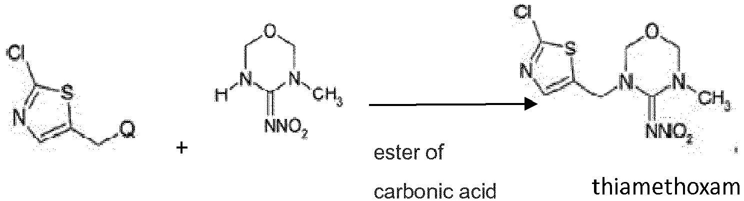

CN 102372702 discloses a method for the preparation of thiamethoxam by the reaction of reacting 3-methyl-N-nitro-1,3,5, oxadiazinan-4-imine and 2-chloro-5-chloromethyl thiazole in a polar aprotic solvent, in particular acetone or acetonitrile. The thiamethoxam product is crystallized from solution in toluene.

WO 01/00623 concerns a method of producing nitroguanidine and nitroenamine derivatives. Thiamethoxam is disclosed as being prepared by the reaction of reacting 3-methyl-N-nitro-1,3,5, oxadiazinan-4-imine and 2-chloro-5-chloromethyl thiazole in dimethyl carbonate. It is described that the thiamethoxam product may be purified by recrystallizing from dimethyl carbonate.

GB 2,514,927 concerns a crystalline form of thiamethoxam and a method for its preparation. In this disclosure, thiamethoxam in a crystalline form is prepared by crystallization from a solution of thiamethoxam in an organic solvent. More particularly, GB 2,514,927 discloses that thiamethoxam can exist in at least one crystalline form. GB 2,514,927 disclose a particular crystalline form of thiamethoxam, designated therein as polymorphic Form A. Organic solvents specified in GB 2,514,927 for forming the crystalline form of thiamethoxam are selected from an alcohol, a glycol, an ether, a ketone, an ester, an amide, a nitrile, an aliphatic or aromatic hydrocarbon, or mixtures thereof. The exemplified solvents are methanol, ethanol, 1-propanol, ethylene glycol, toluene and xylene.

There is a need in the art for efficient methods for the preparation and purification of thiamethoxam, which are simple and can be used on a large scale for industrial manufacture, and which produce a product of high purity, exhibits a high stability and that can be safely utilized.

It has now been found that the stability of the crystalline form of thiamethoxam, in particular the ability of the thiamethoxam to resist photolysis, is dependent upon the solvent used to prepare the crystalline form.

According to the present invention, there is provided the use of a solvent system comprising a C₁ to C₄ alcohol, a substituted benzene derivative bearing two or more C₁ to C₄ alkyl substituents, or a mixture thereof in the preparation of a crystalline form of 3-(2-chloro-1,3-thiazol-5-ylmethyl)-5-methyl-1,3,5-oxadiazinan-4-yledene (nitro)amine (thiamethoxam).

In a further aspect, the present invention provides a method for the preparation of crystalline thiamethoxam exhibiting a high photolytic stability, the method comprising providing a solution of thiamethoxam in a solvent system comprising a C₁ to C₄ alcohol, a substituted benzene derivative bearing two or more C₁ to C₄ alkyl substituents, or a mixture thereof; crystallizing thiamethoxam from the solution; and isolating the resulting crystals.

The present invention relates to the preparation of thiamethoxam in a crystalline form. In a preferred embodiment, thiamethoxam is prepared in a polymorphic form referred to herein as "polymorph Form A".

As discussed above, it has surprisingly been found that the use of a solvent system comprising one or more of the aforementioned solvents produces thiamethoxam in a crystalline form that exhibits a significantly higher resistance to photolysis, compared with the product obtained when using other solvents. The present invention is particularly effective when preparing the polymorph Form A of thiamethoxam.

In another aspect, the present invention provides insecticide compositions comprising thiamethoxam present in a crystalline polymorphic form and prepared using a solvent system as hereinbefore defined.

The insecticide compositions of this aspect of the invention are useful for controlling insects in agricultural and horticultural crops. In one embodiment, the compositions comprise thiamethoxam in crystalline polymorph Form A.

The present invention also relates to methods for combating insects at a locus, the method comprising applying to the locus an insecticidally effective amount of thiamethoxam in a crystalline polymorphic form prepared using a solvent system as hereinbefore defined, in particular the crystalline polymorph Form A of thiamethoxam.

In the method for combating insects, the locus may be a plant to be protected, a region surrounding a plant to be protected, or a seed of the plant.

The present invention also relates to a method for protecting crops, such as agricultural and horticultural crops, including industrial products thereof, such as seeds and fruits, by applying to the crops an effective amount of thiamethoxam in a crystalline polymorphic form prepared using the solvent system as hereinbefore defined, in particular the crystalline polymorph Form A of thiamethoxam.

Solids exist in either amorphous or crystalline forms. In the case of crystalline forms, molecules are positioned in 3-dimensional lattice sites. When a compound recrystallizes from a solution or slurry, it may crystallize with different spatial lattice arrangements, a property referred to as "polymorphism," with the different crystal forms individually being referred to as a "polymorph". Different polymorphic forms of a given substance may differ from each other with respect to one or more physical properties, such as solubility and dissociation, true density, crystal shape, compaction behavior, flow properties, and/or solid state stability.

In the case of a chemical substance that exists in two (or more) polymorphic forms, the unstable forms generally convert to the more thermodynamically stable forms at a given temperature after a sufficient period of time. When this transformation is not rapid, the thermodynamically unstable form is referred to as the "metastable" form. In general, the stable form exhibits the highest melting point, the lowest solubility, and the maximum chemical stability. However, the metastable form may exhibit sufficient chemical and physical stability under normal storage conditions to permit its use in a commercial form. Furthermore, the metastable form, although less stable, may exhibit properties desirable over those of the stable form, such as better formative ability, improved dispersability in water, and the like.

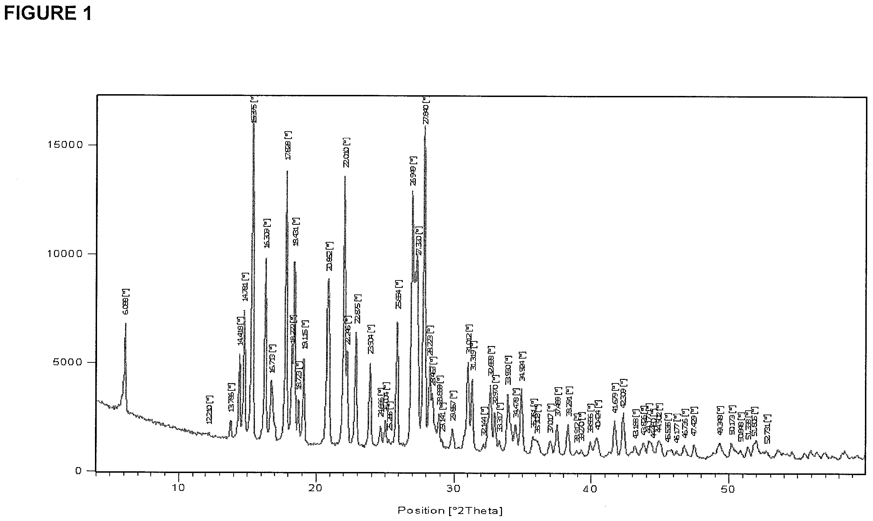

As noted above, the present invention produces thiamethoxam in a crystalline form. The present invention is particularly advantageous when used to prepare polymorph Form A of thiamethoxam. This form exhibits specific spectral characteristics as depicted by the distinct X-ray diffraction patterns and infrared (IR) spectra. For example, as shown in Figure 1, Form A exhibits an X-ray powder diffraction pattern having characteristic peaks (expressed in degrees 2θ +/- 0.2° θ) at one or more of the following positions: 6.09, 15.37, 17.83, 18.43, 20.86, 22.01, 26.95 and 27.84. The X-Ray powder diffraction pattern shown in Figure 1 was collected on a Philips powder diffractometer PW 1050/70 operated at 40 kV and 30 mA using uKα radiation (wavelength equal to 1.54178 Å) and a diffracted beam graphite monochromator. The typical 0 - 20 scan range was 3 - 35° 2 Theta with a step size of 0.05° and a count time of 0.5 seconds per step. The samples were ground using an agate mortar and pestle. The powder so obtained was then pressed into an aluminum sample holder with rectangular cavity with dimensions of 20 mm x 15 mm and a depth of 0.5 mm.

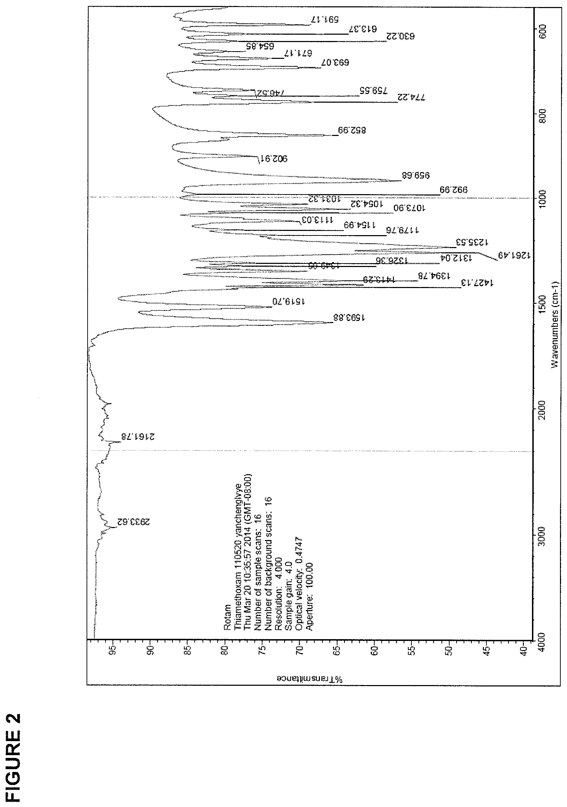

Furthermore, as shown in Figure 2, Form A also exhibits an Infrared (IR) spectrum having characteristic peaks at about 2933.62, 2161.78 and 1593.88 cm⁻¹. The IR spectrum was measured using a Fourier transform infrared (FT-IR) spectrophotometer ReactIR™ 1000 of Mettler Toledo Autochem (ATR method, MCT detector), diamond window, in a DuraSampIIR™ sampling device. The diamond sensor had a standard focusing optic of ZnSe. The powdered samples were compressed in the sampling device and were measured with resolution of 4 cm⁻¹ and 256 scans.

In the present invention, thiamethoxam is prepared by crystallizing thiamethoxam from the solvent system as hereinbefore defined and isolating the resulting crystals.

In one embodiment, the process includes preparing a solution of thiamethoxam in the aforementioned solvent system, preferably by applying heat until dissolution is complete, gradually cooling the solution until crystals appear, and isolating the crystals. Generally, cooling to room temperature is sufficient, however, the solution can be cooled to lower temperatures, for example 0°C, 5°C, 10°C, 15°C and the like. Gradual cooling is typically achieved, for example by removing the heat and allowing the solution to cool under ambient conditions.

The formation of crystals from the solution of thiamethoxam may be seeded with seed crystals, for example Form A seed crystals, in order to induce crystallization, as known in the art.

Any suitable crystallization techniques can be employed in the present invention, including batch crystallization, semi-batch crystallization and continuous crystallization.

The thiamethoxam starting material used in the present invention can be prepared by methods known in the art. One preferred route to forming thiamethoxam is the reaction of 3-methyl-N-nitro -1,3,5, oxadiazinan-4-imine and 2-chloro-5-chloromethyl thiazole, according to the following general reaction scheme:

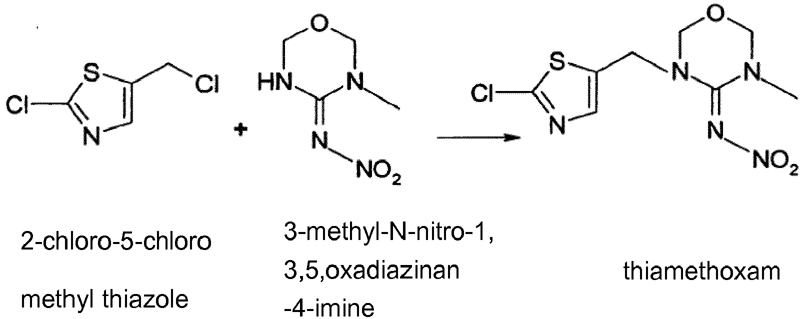

The above reaction is conducted in the presence of a solvent or diluent, a phase transfer catalyst and a base, preferably the solvent or diluent being a polar aprotic solvent, the phase transfer catalyst being a quaternary ammonium salt and the base being a carbonate. DMF is a particularly suitable solvent for conducting the above reaction. A suitable phase transfer catalyst is triethyl benzyl ammonium chloride (TEBA). The base used is preferably potassium carbonate.

In the present invention, a solvent is used to prepare thiamethoxam in a crystalline form. In particular, the present invention uses a solvent system comprising a C₁ to C₄ alcohol, a substituted benzene derivative bearing two or more C₁ to C₄ alkyl substituents, or a mixture thereof.

The solvent system may comprise one or more C₁ to C₄ alcohols. Preferred alcohols are C₁, C₂ and C₃ alcohols. The alcohol may be a mono-alcohol, preferably methanol, ethanol or propanol. Mono-alcohols having three or more carbon atoms are preferably 1-alkanols, with 1-propanol one preferred alcohol. Alternatively, the alcohol may be a polyol, preferably a glycol. Preferred polyols are C₂, C₃ and C₄ polyols, especially glycols, with ethylene glycol being on preferred polyol.

The solvent system may comprise a substituted benzene derivative bearing two or more C₁ to C₄ alkyl substituents, preferably two or more C₁ to C₃ alkyl substituents, more preferably two or more substituents selected from methyl or ethyl moieties. The two or more alkyl substituents are preferably the same moiety. Methyl is a particularly preferred alkyl substituent. Preferred benzene derivatives are those have 2 to 4 substituents, more preferably 2 to 3 substituents, with 2 substituents being especially preferred. Xylene is a particularly preferred benzene derivative.

The present invention has been found to be particularly effective when the solvent system comprises methanol, ethanol, propanol, especially 1-propanol, ethylene glycol, xylene or mixtures of two or more thereof.

The solvent system may comprise two more of the aforementioned solvents. In many preferred embodiments, the solvent system consists essentially of a single solvent.

Thiamethoxam in a crystalline form prepared using the present invention may be formulated and incorporated into insecticidal compositions using techniques known in the art. These are summarized as follows:

Aqueous use forms can be prepared from emulsion concentrates, pastes or wettable powders (sprayable powders, oil dispersions) by adding water. To prepare emulsions, pastes or oil dispersions, the active ingredients, as such or dissolved in an oil or solvent, can be homogenized in water by means of stirring with a wetter, tackifier, dispersant or emulsifier. However, it is also possible to prepare concentrates composed of an active substance, wetter, tackifier, dispersant or emulsifier and, if appropriate, solvent or oil, and such concentrates are suitable for dilution with water.

The active compound concentrations in the ready-to-use preparations can be varied within relatively wide ranges. In general, suitable concentrations are from 0.0001 to 10%, preferably from 0.01 to 1% by weight.

The composition may contain thiamethoxam as the sole active ingredient. Alternatively, the composition may further comprise one or more other active components that are compatible for combination with thiamethoxam.

The following are examples of formulations of the thiamethoxam products of the present invention:
1. Products for dilution with water for foliar applications may be prepared as follows. The formulations may also be applied to seeds, either with or without dilution, for seed treatment purposes.

### A) Water-Soluble Concentrates (SL, LS)

10 parts by weight of the active compound(s) are dissolved in 90 parts by weight of water or a water-soluble solvent. Wetters or other auxiliaries may be added. The active compound(s) dissolves upon dilution with water, whereby a formulation with 10% (w/w) of active compound(s) is obtained.

### B) Dispersible Concentrates (DC)

20 parts by weight of the active compound(s) are dissolved in 70 parts by weight of cyclohexanone with the addition of 10 parts by weight of a dispersant, for example polyvinylpyrrolidone. Dilution with water gives a dispersion, whereby a formulation with 20% (w/w) of active compound(s) is obtained.

### C) Emulsifiable Concentrates (EC)

15 parts by weight of the active compound(s) are dissolved in 80 parts by weight of xylene with the addition of calcium dodecylbenzenesulfonate and castor oil ethoxylate (in each case 5 parts by weight). Dilution with water gives an emulsion, whereby a formulation with 15% (w/w) of active compound(s) is obtained.

### D) Emulsions (EW, EO, ES)

25 parts by weight of the active compound(s) are dissolved in 35 parts by weight of xylene with the addition of calcium dodecylbenzenesulfonate and castor oil ethoxylate (in each case 5 parts by weight). This mixture is introduced into 30 parts by weight of water by means of an emulsifier machine (for example an Ultraturrax) and formed into a homogeneous emulsion. Dilution with water gives an emulsion, whereby a formulation with 25% (w/w) of active compound(s) is obtained.

### E) Suspensions (SC, OD, FS)

In an agitated ball mill, 20 parts by weight of the active compound(s) are comminuted with the addition of 10 parts by weight of dispersants, wetters and 70 parts by weight of water or of an organic solvent to give a fine active compound(s) suspension. Dilution with water gives a stable suspension of the active compound(s), whereby a formulation with 20% (w/w) of active compound(s) is obtained.

### F) Water-Dispersible Granules and Water-Soluble Granules (WG, SG)

50 parts by weight of the active compound(s) are ground finely with the addition of 50 parts by weight of dispersants and wetters and formed as water-dispersible or water-soluble granules by means of technical appliances (for example extrusion, spray tower, fluidized bed). Dilution with water gives a stable dispersion or solution of the active compound(s), whereby a formulation with 50% (w/w) of active compound(s) is obtained.

### G) Water-Dispersible Powders and Water-Soluble Powders (WP, SP, SS, WS)

75 parts by weight of the active compound(s) are ground in a rotor-stator mill with the addition of 25 parts by weight of dispersants, wetters and silica gel. Dilution with water gives a stable dispersion or solution of the active compound(s), whereby a formulation with 75% (w/w) of active compound(s) is obtained.

### H) Gel-Formulation (GF) (for Seed Treatment Purposes Only)

In an agitated ball mill, 20 parts by weight of the active compound(s) are comminuted with the addition of 10 parts by weight of dispersants, 1 part by weight of a gelling agent/wetters and 70 parts by weight of water or of an organic solvent to give a fine active compound(s) suspension. Dilution with water gives a stable suspension of the active compound(s), whereby a formulation with 20% (w/w) of active compound(s) is obtained.
2. Products to be applied undiluted for foliar applications may be prepared as follows. For seed treatment purposes, such products may be applied to the seed diluted.

### I) Dustable Powders (DP, DS)

5 parts by weight of the active compound(s) are ground finely and mixed intimately with 95 parts by weight of finely divided kaolin. This formulation gives a dustable product having 5% (w/w) of active compound(s).

### J) Granules (GR, FG, GG, MG)

0.5 parts by weight of the active compound(s) is ground finely and associated with 95.5 parts by weight of carriers, whereby a formulation with 0.5% (w/w) of active compound(s) is obtained. Current methods for preparing granules include extrusion, spray-drying or use of a fluidized bed. This gives granules to be applied undiluted for foliar use.

### K) Microcapsulation, ME

0.5 parts by weight of the active compound(s) is ground finely and associated with 95.5 parts by weight of a mixture of polyurea, crosslinker, and carriers, whereby a formulation with 0.5% (w/w) of active compound(s) is obtained. This gives a microencapsulation product having 5% (w/w) of active compound(s), in which the active ingredient is encapsulated within microcapsules having a polymer shell.

### L) Microcapsulation Granules, MEG

0.5 part by weight of the active compound(s) is ground finely and associated with 95.5 parts by weight of polyurea, crosslinker, a solid carrier and a binder to form a mixture, forming granules from the resulting mixture; applying a composition comprising a binder to coat the granules; and drying the thus coated granules. This procedure gives a microencapsulated active ingredient within granules having 5% (w/w) of active compound(s).

Embodiments of the present invention will now be illustrated by the following specific examples.

### EXAMPLES

### Example 1: Synthesis of Thiamethoxam

### 1.1 Preparation of 3,6-dihydro-3-methyl-N-nitro-2H-1,3,5,oxadiazin-4-amine

3,6-dihydro-3-methyl-N-nitro-2H-1,3,5,oxadiazin-4-amine was prepared using the following reaction scheme:

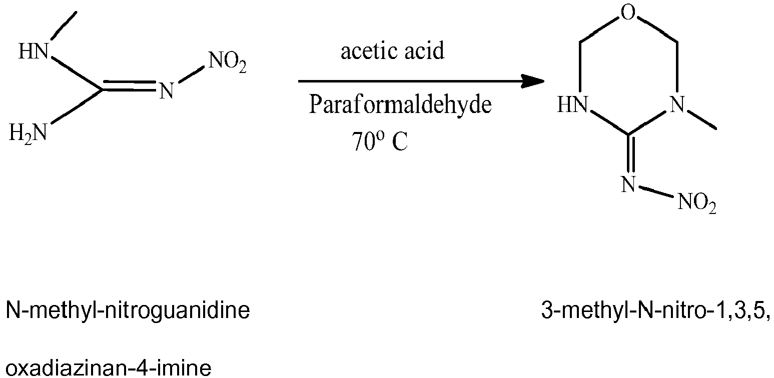

100 kg of N-methyl-nitroguanidine and 64 kg of Paraformaldehyde were charged to a 1000 L reactor. 350 kg of acetic acid was added, after which the resulting mixture was heated to 70°C and held at this temperature for 6 hours. Thereafter, the solvent (acetic acid) was removed by distillation under vacuum. 175 kg of 10% NaOH aqueous solution was added with stirring, after which the resulting mixture was cooled and stirred for 30 minutes. The resulting mixture was discharged into a centrifuge for separation. The resulting cake was dried using a Biconical dryer, yielding 98 kg of 3-methyl-N-nitro-1,3,5,oxadiazinan-4-imine as a white powder (purity 97%, yield 71.5%).

### 1.2 Preparation of 2-chloro-allyl thioisocyanide

2-chloro-allyl thioisocyanide was prepared using the following reaction scheme:

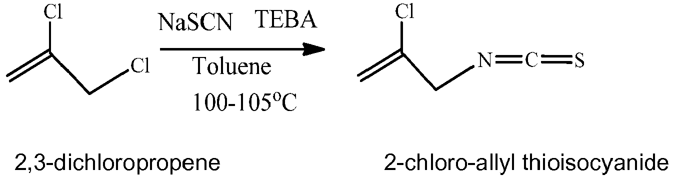

In a 500 L reactor, 60.5 kg of 2,3-dichloropropene was mixed with 135 kg of toluene, 0.5 kg of TEBA (Triethyl benzyl ammonium chloride, as catalyst) and 44.1 kg of sodium thiocyanate. The resulting mixture was heated under reflux (about 100 to 105°C) for 1.5 hours and then cooled to room temperature. 50 kg of water was added with stirring for 15 minutes, after which the mixture was allowed to stand and the phases allowed to stratify. Toluene from the organic phase was removed under vacuum and the residue was distilled under high vacuum using a Roots Vacuum Pump. 65 kg of distillate, 2-chloro-allyl thioisocyanid, were recovered as a yellow oil liquid (yield 80%, purity 90%).

### 1.3 Preparation of 2-chloro-5-(chloromethyl)thiazole

2-chloro-5-(chloromethyl) thiazole was prepared using the following reaction procedure:

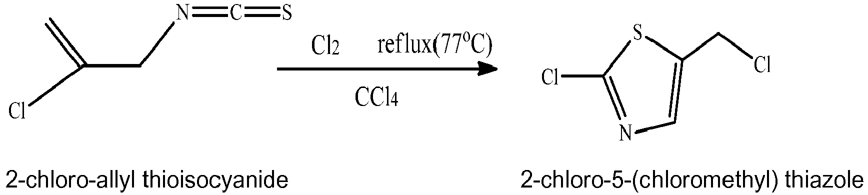

65 kg of 2-chloro-allyl thioisocyanide was mixed with 140 kg of carbon tetrachloride in a 500 L enamel reactor. 35 kg chlorine was bubbled into the mixture over a period of one hour, the resulting mixture heated to reflux (77°C) for 3 hours, and then cooled to room temperature. Carbon tetrachloride was removed by distillation. 59 kg of dichloromethane was added and stirred until the residue was dissolved, after which the solution was washed with 86 kg of saturated NaHCO₃ solution and 40 kg of water. The resulting mixture was dried with anhydrous MgSO₄, after which dichloromethane was removed by distillation under vacuum. Finally, the reaction product was isolated by distillation under high vacuum using a Roots vacuum pump, to yield 61 kg of 2-chloro-5-(chloromethyl) thiazole as a yellow liquid (yield 84%, purity 96%).

### 1.4 Preparation of Thiamethoxam

Thiamethoxam was prepared by the following reaction scheme:

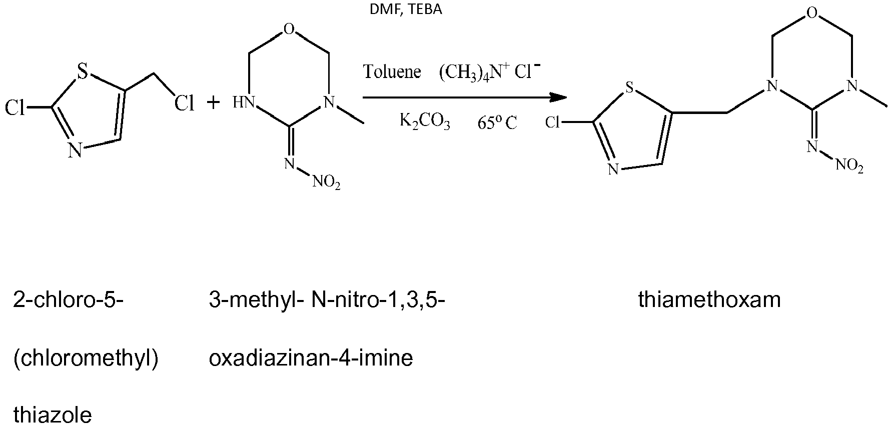

47.5 kg of 3-methyl-N-nitro-1,3,5-oxadiazinan-4-imine and 50 kg of 2-chloro-5-(chloromethyl) thiazole were fed to a 1000 L enamel reactor containing 350 kg of dimethyl formamide (DMF). The resulting mixture was heated and the temperature held at about 65°C. Thereafter, 82 kg of potassium carbonate and 1 kg of triethyl benzyl ammonium chloride (TEBA, as catalyst) were added to the reactor over a period of from 20 to 40 minutes. The resulting mixture was allowed to react for from 4 to 5 hours, after which the mixture was allowed to cool to room temperature. 280 kg of water was added to the reactor, the mixture stirred for 15 minutes and the pH adjusted to between 6 and 7 with 32% hydrochloric acid. The resulting mixture was stirred severely and heated to 65°C. The resulting mixture was allowed to stand for 30 minutes, after which the water phase was discharged and extracted 3 times with dichloromethane (DCM) (100 kg x 3). The organic phases were combined and DCM removed from the mixture by distillation under vacuum. The mixture was dried using a Biconical dryer at 40°C, to yield crude thiamethoxam.

### Example 2: Preparation of Crystalline Thiamethoxam using Methanol

2 g of thiamethoxam as prepared in Example 1 was heated in 10 g of methanol until complete dissolution was achieved. The resulting solution was heated under reflux for 30 to 60 minutes, then cooled to room temperature. The resulting mixture was filtered to isolate a solid. The resulting solid was washed with methanol several times and dried under high vacuum to give crystals of pure thiamethoxam technical (purity: 98%).

The crystals were characterized as being of thiamethoxam polymorph Form A using both infrared (IR) spectrometry and X-ray diffraction.

Form A thiamethoxam has the X-ray powder diffractogram shown in Figure 1 with the reflexes listed in Table 1 below.

**Table 1**

| 2 θ and d-value of modification I | |
|---|---|
| 2 θ (°) | d (Å) |
| 6.09 ± 0.2 | 14.52 ± 0.05 |
| 15.37 ± 0.2 | 5.76 ± 0.03 |
| 17.83 ± 0.2 | 4.98 ± 0.03 |
| 18.43 ± 0.2 | 4.81 ± 0.02 |
| 20.86 ± 0.2 | 4.26 ± 0.02 |
| 22.01 ± 0.2 | 4.04 ± 0.02 |
| 26.95 ± 0.2 | 3.31 ± 0.02 |
| 27.84 ± 0.2 | 3.20 ± 0.02 |

The IR spectrum of Form A thiamethoxam is set out in Figure 2. The IR spectrum exhibits characteristic peaks at 2933.62, 2161.78 and 1593.88 cm⁻¹.

### Example 3: Preparation of Crystalline Thiamethoxam using Xylene

2 g of thiamethoxam prepared as described in Example 1 was dissolved in 10 g of xylene while applying low heating over a heating plate. The resulting mixture was heated under reflux for 30 to 60 minutes, then cooled to room temperature. The resulting mixture was filtered to isolate a solid. After filtration, the solid was washed with xylene several times and dried under high vacuum to give crystals of pure thiamethoxam technical (purity: 97%).

The crystals were characterized as being thiamethoxam Form A using infrared spectrometry and X-ray diffraction, as described in Example 2.

### Example 4: Preparation of Crystalline Thiamethoxam using Ethylene Glycol

2 g of thiamethoxam prepared as described in Example 1 and 10 g of ethylene glycol were heated until complete dissolution of the thiamethoxam was reached. The resulting mixture was heated under reflux for 30 to 60 minutes, then cooled to room temperature. The resulting mixture was filtered to isolate a solid. After filtration, the solid was washed with ethylene glycol several times and dried under high vacuum to give crystals of pure thiamethoxam technical (purity: 96%).

The crystals were characterized as being thiamethoxam Form A using infrared spectrometry and X-ray diffraction, as described in Example 2.

### Example 5: Preparation of Crystalline Thiamethoxam using Ethanol

2 g of thiamethoxam as prepared in Example 1 and 10 g of ethanol were heated until complete dissolution of the thiamethoxam was achieved. The resulting mixture was heated under reflux for 30 to 60 minutes, then cooled to room temperature. The resulting mixture was filtered to isolate a solid. After filtration, the solid was washed with ethanol several times and dried under high vacuum to give crystals of pure thiamethoxam technical (purity: 96%).

The crystals were characterized as being thiamethoxam Form A using infrared spectrometry and X-ray diffraction, as described in Example 2.

### Example 6: Preparation of Crystalline Thiamethoxam using Propanol

2 g of thiamethoxam prepared as described in Example 1 and 10 g of 1-propanol were heated until complete dissolution of the solid thiamethoxam was achieved. The resulting mixture was heated under reflux for 30 to 60 minutes, then cooled to room temperature. The resulting mixture was filtered to isolate a solid. After filtration, the solid was washed with 1-propanol several times and dried under high vacuum to give crystals of pure thiamethoxam technical (purity: 97%).

The crystals were characterized as being thiamethoxam Form A using infrared spectrometry and X-ray diffraction, as described in Example 2.

### Comparative Example A: Preparation of Crystalline Thiamethoxam using Toluene

2 g of Thiamethoxam prepared as described in Example 1 and 10 g of toluene were heated until complete dissolution was achieved. The resulting mixture was heated under reflux for 30 to 60 minutes, then cooled to room temperature. The resulting mixture was filtered to isolate a solid. The resulting solid was washed with toluene several times and dried under high vacuum to give crystals of pure thiamethoxam technical (purity: 97%).

The crystals were characterized as being thiamethoxam Form A using infrared spectrometry and X-ray diffraction, as described in Example 2.

### Photolysis Stability Tests

The samples of crystalline thiamethoxam prepared in Examples 2 to 6 and in Comparative Example A were subjected to a test to determine their photolysis stability, using the following procedure:
20 mL of the thiamethoxam aqueous solution (50mg/L) was added to a quartz tube. The tube was continuously irradiated with UV light from a UV lamp. Aliquots (100 µL) of the solution were removed from the tube at intervals of 10 minutes. The concentration of thiamethoxam in each aliquot was determined by gas chromatography (GC) and mass spectrometry (MS).

The results are summarized in Table 2 below.

**Table 2**

| Solvent | | Thiamethoxam (%wt) | | |
|---|---|---|---|---|
| | 0 minutes | 10 minutes | 20 minutes | 30 minutes |
| Crude Thiamethoxam | 100 | 70 | 43 | 17 |
| Methanol | 99 | 96 | 90 | 73 |
| Xylene | 100 | 95 | 91 | 75 |
| Ethylene glycol | 98 | 93 | 88 | 73 |
| Ethanol | 100 | 96 | 90 | 71 |
| 1-Propanol | 100 | 95 | 93 | 70 |
| Toluene | 100 | 90 | 79 | 55 |

As can be seen from the data in Table 2 above, the crystalline thiamethoxam obtained in each of Examples 2 to 6 exhibited a very high resistance to photolysis compared with the crude thiamethoxam product of Example 1. In addition, the results show that the preparation of crystalline thiamethoxam by crystallization from a solution in toluene yielded a crystalline product having a poor photolytic stability.

## Claims

1. Use of a solvent system comprising a C₁ to C₄ alcohol, a substituted benzene derivative bearing two or more C₁ to C₄ alkyl substituents, or a mixture thereof in the preparation of a crystalline form of 3-(2-chloro-1,3-thiazol-5-ylmethyl)-5-methyl-1,3,5-oxadiazinan-4-yledene (nitro)amine (thiamethoxam).

2. The use according to claim 1, wherein the crystalline form of exhibits an X-ray powder diffraction pattern having characteristic peaks (expressed in degrees 2θ +/-0.2° θ) at one or more of the following positions: 6.09, 15.37, 17.83, 18.43, 20.86, 22.01, 26.95 and 27.84.

3. The use according to either of claims 1 or 2, wherein the crystalline form exhibits an Infrared (IR) spectrum having characteristic peaks at about 2933.62, 2161.78 and 1593.88 cm⁻¹.

4. The use according to any preceding claim, wherein the solvent system comprises a C₁ to C₄ alcohol, preferably wherein the alcohol is a mono-alcohol, more preferably methanol, ethanol or propanol; or preferably wherein the alcohol is a diol, more preferably ethylene glycol.

5. The use according to any preceding claim, wherein the solvent system comprises a substituted benzene derivative bearing two or more C₁ to C₄ alkyl substituents, preferably a substituted benzene derivative bearing two methyl or ethyl substituents, more preferably xylene.

6. The use according to any preceding claim, wherein the solvent system is cooled to a temperature of from 0 to 15°C.

7. The use according to any preceding claim, wherein the solvent system is seeded with crystals of a crystalline form of 3-(2-chloro-1,3-thiazol-5-ylmethyl)-5-methyl-1,3,5-oxadiazinan -4-yledene (nitro)amine (thiamethoxam), preferably a crystalline form according to either of claims 2 or 3.

8. An insecticide composition comprising a crystalline form of 3-(2-chloro-1,3-thiazol-5-ylmethyl)-5-methyl-1,3,5-oxadiazinan-4-yledene (nitro)amine (thiamethoxam) prepared according to the use of any preceding claim.

9. A method for combating insects at a locus, the method comprising applying to the locus a crystalline form of 3-(2-chloro-1,3-thiazol-5-ylmethyl)-5-methyl-1,3,5-oxadiazinan -4-yledene (nitro)amine (thiamethoxam) prepared by the use according to any of claims 1 to 7 or a composition according to claim 8.

10. The method according to claim 9, wherein the locus is a plant to be protected, a region surrounding a plant to be protected or a seed of a plant to be protected.

11. A method of protecting crops, the method comprising applying to the crops an effective amount of a crystalline form of 3-(2-chloro-1,3-thiazol-5-ylmethyl)-5-methyl-1,3,5-oxadiazinan-4-yledene (nitro)amine (thiamethoxam) prepared by the use according to any of claims 1 to 7 or of a composition according to claim 8.

12. The use of a crystalline form of 3-(2-chloro-1,3-thiazol-5-ylmethyl)-5-methyl-1,3,5-oxadiazinan-4-yledene (nitro)amine (thiamethoxam) prepared by the use according to any of claims 1 to 7 in combating insects.
